# EUROPÄISCHE PATENTSCHRIFT

(11) **EP 1 107 946 B1**
(45) Veröffentlichungstag und Bekanntmachung des Hinweises auf die Patenterteilung: **04.12.2002**
(21) Anmeldenummer: 99944516.6
(22) Anmeldetag: 26.08.1999
(51) Int. Cl.: C07C 233/58, A01N 53/00, A01N 37/18

(54) **CYCLOALKYLCARBONSÄUREAMIDE, DEREN HERSTELLUNG UND VERWENDUNG ALS FUNGIZIDE IM AGRARBEREICH**
CYCLOALKYL CARBOXYLIC ACID AMIDES, THEIR PRODUCTION AND THEIR USE AS FUNGICIDES IN AGRICULTURE
AMIDES D'ACIDES CYCLOALKYLCARBOXYLIQUES, LEUR FABRICATION ET LEUR UTILISATION COMME FONGICIDES EN AGRICULTURE

(30) Priorität: 01.09.1998 DE 19839690
(43) Veröffentlichungstag der Anmeldung: 20.06.2001
(73) Patentinhaber: BASF AKTIENGESELLSCHAFT, 67056 Ludwigshafen (DE)
(72) Erfinder: ROSE, Ingo, D-68159 Mannheim (DE); WETTERICH, Frank, D-67157 Wachenheim (DE); EICKEN, Karl, D-67157 Wachenheim (DE); RHEINHEIMER, Joachim, D-67063 Ludwigshafen (DE); LORENZ, Gisela, D-67434 Neustadt (DE); AMMERMANN, Eberhard, D-64646 Heppenheim (DE); GROTE, Thomas, D-67105 Schifferstadt (DE); STRATHMANN, Siegfried, D-67117 Limburgerhof (DE)
(86) Internationale Anmeldenummer: EP9906251
(87) Internationale Veröffentlichungsnummer: WO00012465

(56) Entgegenhaltungen:
- EP-A- 0 170 842
- EP-A- 0 257 448
- EP-A- 0 341 475
- WO-A-97/35838
- WO-A-98/41499
- WO-A-99/31048
- STRAUB R ET AL: "NEW CHIRAL COVALENTLY BONDED, -DONOR STATIONARY PHASES FOR HIGH-PERFORMANCE LIQUID CHROMATOGRAPHY, BASED ON DERIVATIVES OF OPTICALLY ACTIVE 1-(1-NAPHTHYL)ETHYLAMINE" JOURNAL OF CHROMATOGRAPHY,NL,ELSEVIER SCIENCE PUBLISHERS B.V. AMSTERDAM, Bd. 585, Nr. 2, 1. November 1991 (1991-11-01), Seiten 195-206, XP000231966 ISSN: 0021-9673
- HANS-GEORG KUBALL ET AL: "Helical Twisting Power and Circular Dichroism as Chirality Observations: the Intramolecular and Intermolecular Chiratity Transfer" CHIRALITY,US,WILEY-LISS, NEW YORK, Bd. 9, Nr. 5/06, 1. Januar 1997 (1997-01-01), Seiten 407-423, XP002095750 ISSN: 0899-0042

## Beschreibung

Die vorliegende Erfindung betrifft neue Cycloalkylcarbonsäureamide der allgemeinen Formel I 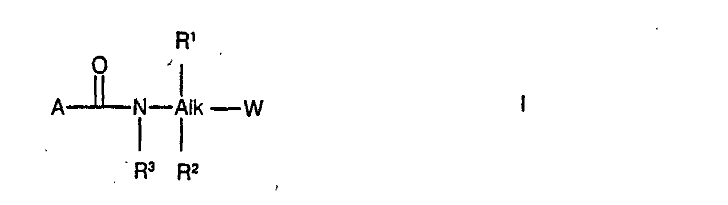 in der die Substituenten die folgende Bedeutung haben:
- A: C₃-C₆-Cycloalkyl, welches einen oder mehrere Substituenten ausgewählt aus der Gruppe bestehend aus Halogen, Cyano, C₁-C₆-Alkyl, C₂-C₆-Alkenyl, C₁-C₆-Alkoxy oder C₁-C₆-Alkylthio tragen kann;
- Alk: geradkettiges oder verzweigtes C₁-C₆-Alkylen;
- R¹: C₁-C₆-Alkyl oder C₂-C₆-Alkenyl, wobei diese Reste partiell oder vollständig halogeniert sein und/oder eine oder zwei der folgenden Gruppen tragen können: C₁-C₄-Alkoxy, C₁-C₄-Halogenalkoxy, C₁-C₄-Alkylthio, C₁-C₄-Alkoxycarbonyl, C₃-C₆-Cycloalkyl und Phenyl, wobei das Phenyl partiell oder vollständig halogeniert sein und/oder einen bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy, C₁-C₄-Alkylthio, C₃-C₆-Cycloalkyl oder Heterocyclyl;
- R², R³: Wasserstoff, C₁-C₆-Alkyl oder C₂-C₆-Alkenyl, wobei diese Reste partiell oder vollständig halogeniert sein können;
- W: kondensiertes bicyclisches Ringsystem mit jeweils sechs Ringatomen, wobei ein oder zwei C-Ringatome durch N-Atome ausgetauscht sein können, und wobei diese Ringsysteme eine bis drei der folgenden Gruppen tragen können: Nitro, Halogen, Cyano, C₁-C₄-Alkyl, C₁-C₄-Halogenalkyl, C₁-C₄-Alkoxy, C₁-C₄-Halogenalkoxy, C₁-C₄-Alkylthio, C₃-C₆-Cycloalkyl und C₁-C₄-Alkoxycarbonyl;
sowie deren landwirtschaftlich einsetzbaren Salze.

In WO 99/31408 werden Cyclobutylcarbonsäureamide beschrieben. Aus Straub et al., J. of Chromatography, Vol 585 (1991), S. 195-206 und Kuball et al., Chirality, Vol 9 (1997), S. 407-423 sind Cyclohexylcarbonsäureamide bekannt. WO 97/35838 offenbart bi- und tricyclisch substituierte Carbonsäureamide. In EP 0 170 842 werden N-Benzylcyclopropancarboxamid-Derivate und deren Verwendung als Fungizide in der Landwirtschaft beschrieben. Bei den dort beschriebenen Verbindungen handelt es sich um Cyclopropylcarbonsäureamide, die am Amidteil eine Phenylalkylgruppe tragen, und die in 2-Stellung des Cyclopropanringes durch zwei Chloratome substituiert sind. In US 4,988,734 werden die entsprechenden N-(R)-(1-Phenylethyl)-1-alkyl-2,2-dichlor-cyclopropan-carboxamid-Stereoisomere beschrieben. In EP 0 257 488 werden optisch aktive Cyclopropylcarboxamide, welche im Amidteil eine Benzylgruppe tragen, offenbart. Ferner sind in US 5,034,408 weitere 2,2-Dichlorcyclopropan-carboxamide beschrieben, die im Carboxamidteil eine zweite Carboxamid-Gruppe enthalten. Aus PCT/EP98/01031 sind ferner α-Halogen- und α-Cyan-substituierte Carbonsäureamide mit fungizider Wirkung bekannt.

Da die fungiziden Eigenschaften der bekannten Verbindungen hinsichtlich ihrer Aktivität gegen Schadpilze, wie z.B. *Pyricularia oryzae,* nicht immer voll befriedigen, war es die Aufgabe der vorliegenden Erfindung, neue gegen Schadpilze wirksame Carbonsäureamide zu finden.

Demgemäß wurden die eingangs definierten Cycloalkylalkancarbonsäureamide I gefunden. Desweiteren wurden Verfahren zur Herstellung der Verbindungen I und die zu deren Herstellung benötigten Zwischenprodukte der Formel II gefunden. Es wurden Mittel gefunden, die die Verbindungen I enthalten, Verfahren zur Bekämpfung von Schadpilzen mit den Verbindungen I und schließlich die Verwendung der Verbindungen I zur Bekämpfung von Schadpilzen.

Die Verbindungen der Formel I können je nach Substitutionsmuster ein oder mehrere Chiralitätszentren enthalten. Sie liegen dann als Enantiomeren- bzw. Diastereomerengemische vor. Gegenstand der Erfindung sind sowohl die reinen Enantiomeren oder Diastereomeren als auch deren Gemische.

Bei der eingangs angegebenen Definition der Verbindungen I wurden für die Reste R¹ bis R³ und A Sammelbegriffe verwendet, die für individuelle Aufzählungen der einzelnen Gruppenmitglieder stehen. Die Reste Alkyl, Alkylthio, Alkoxy, Alkoxycarbonyl und Alkenyl können in allen genannten Fällen geradkettig oder verzweigt sein.

Die Angabe "partiell oder vollständig halogeniert" soll zum Ausdruck bringen, daß in den derart charakterisierten Gruppen die Wasserstoffätome zum Teil oder vollständig durch gleiche oder verschiedene Halogenatome ersetzt sein können. Die Bedeutung Halogen steht jeweils für Fluor, Chlor, Brom oder Jod. verschiedene Halogenatome ersetzt sein können. Die Bedeutung Halogen steht jeweils für Fluor, Chlor, Brom oder Jod.

Für den Fall, daß W einen Naphthylring darstellt, bei dem ein oder zwei Kohlenstoffringatome durch Stickstoffatome ersetzt sind, versteht man darunter Chinolin-, Isochinolin- oder Naphthyridin-Systeme.

Ferner bedeuten beispielsweise in allen jeweils genannten Fällen:
- C₁-C₄-Alkyl, sowie die Alkylteile von C₁-C₄-Alkylthio:
   Methyl, Ethyl, Propyl, 1-Methylethyl, Butyl, 1-Methylpropyl, 2-Methylpropyl und 1,1-Dimethylethyl;
- C₁-C₆-Alkyl;
   C₁-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₄-Halogenalkyl:
   einen C₁-C₄-Alkylrest wie voranstehend genannt, der partiell oder vollständig durch Fluor, Chlor, Brom und/oder Jod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Jodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₄-Alkoxy sowie die Alkoxyteile von C₁-C₄-Alkoxycarbonyl:
   Methoxy, Ethoxy, Propoxy, 1-Methylethoxy, Butoxy, 1-Methylpropoxy, 2-methylpropoxy und 1,1-Dimethylethoxy;
- C₁-C₄-Halogenalkoxy: einen C₁-C₄-Alkoxyrest wie voranstehend genannt, der partiell oder vollständig durch Fluor, Chlor, Brom und/oder Jod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-Jodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₂-C₆-Alkenyl: Ethylen, Prop-1-en-1-yl, Prop-2-en-1-yl, 1-Methylethenyl, Buten-1-yl, Buten-2-yl, Buten-3-yl, 1-Methyl-prop-1-en-1-yl, 2-Methyl-prop-1-en-1-yl, 1-Methylprop-2-en-1-yl und 2-Methyl-prop-2-en-1-yl, Penten-1-yl, Penten-2-yl, Penten-3-yl, Penten-4-yl, 1-Methylbut-1-en-1-yl, 2-Methyl-but-1-en-1-yl, 3-Methylbut-1-en-1-yl, 1-Methyl-but-2-en-1-yl, 2-Methylbut-2-en-1-yl, 3-Methyl-but-2-en-1-yl, 1-Methylbut-3-en-1-yl, 2-Methyl-but-3-en-1-yl, 3-Methylbut-3-en-1-yl, 1,1-Dimethyl-prop-2-en-1-yl, 1,2-Dimethylprop-1-en-1-yl, 1,2-Dimethyl-prop-2-en-1-yl, 1-Ethylprop-1-en-2-yl, 1-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-1-en-1-yl, 2-Methyl-pent-1-en-1-yl, 3-Methylpent-1-en-1-yl, 4-Methyl-pent-1-en-1-yl, 1-Methylpent-2-en-1-yl, 2-Methyl-pent-2-en-1-yl, 3-Methylpent-2-en-1-yl, 4-Methyl-pent-2-en-1-yl, 1-Methylpent-3-en-1-yl, 2-Methyl-pent-3-en-1-yl, 3-Methylpent-3-en-1-yl, 4-Methyl-pent-3-en-1-yl, 1-Methylpent-4-en-1-yl, 2-Methyl-pent-4-en-1-yl, 3-Methylpent-4-en-1-yl, 4-Methyl-pent-4-en-1-yl, 1,1-Dimethylbut-2-en-1-yl, 1,1-Dimethyl-but-3-en-1-yl, 1,2-Dimethylbut-1-en-1-yl, 1,2-Dimetyl-but-2-en-1-yl, 1,2-Dimethylbut-3-en-1-yl, 1,3-Dimetyl-but-1-en-1-yl, 1,3-Dimethylbut-2-en-1-yl, 1,3-Dimetyl-but-3-en-1-yl, 2,2-Dimethylbut-3-en-1-yl, 2,3-Dimetyl-but-1-en-1-yl, 2,3-Dimethylbut-2-en-1-yl, 2,3-Dimetyl-but-3-en-1-yl, 3,3-Dimethylbut-1-en-1-yl, 3,3-Dimetyl-but-2-en-1-yl, 1-Ethylbut-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethylbut-3-en-1-yl, 1,1,2-Trimethyl-prop-2-en-1-yl, 1-Ethyl-1-methyl-prop-2-en-1-yl, 1-Ethyl-2-methylprop-1-en-1-yl und 1-Ethyl-2-methyl-prop-2-en-1-yl;
- C₃-C₆-Cycloalkyl: Cyclopropyl, Cyclobutyl, Cyclopentyl, Cyclohexyl; vorzugsweise Cyclopropyl;
- C₁-C₆-Alkylen: geradekettige oder verzweigte Alkylengruppe mit 1 - 6 C-Atomen, wie z.B. Methylen, 1,1-Ethylen, 1,2-Ethylen, 1,1-Propylen, 1,2-Propylen, 1,3-Propylen, 1,1-Butylen, 1,2-Butylen, 1,3-Butylen, 1,4-Butylen; insbesondere Methylen.
- C₁-C₆-Alkyl- bzw. C₂-C₆-Alkenyl, wobei diese Reste partiell oder vollständig halogeniert sein können: Die entsprechenden unter C₁-C₆-Halogenalkyl genannten Reste, wobei im Fall der Alkenylreste die zuvor in der Definition von Alkenyl genannten Reste in Frage kommen.

Im Sinne der vorliegenden Erfindung sind Cycloalkylcarbonsäureamide I mit folgenden Substituenten bevorzugt, wobei die Bevorzugung jeweils für sich allein oder in Kombination mit den übrigen Definitionen zu sehen ist:
R¹ bedeutet bevorzugt C₁-C₆-Alkyl, wie z.B. Methyl oder Ethyl. R² bedeutet bevorzugt Wasserstoff oder C₁-C₆-Alkyl, wie z.B. Methyl oder Ethyl. Besonders bevorzugt sind Verbindungen I, bei denen R¹ für Methyl und R² für Wasserstoff steht.
W bedeutet bevorzugt 1-Naphthyl oder 2-Naphthyl.
Alk bedeutet bevorzugt die Methylengruppe, wobei Verbindungen vom Typ -C(R³) (R⁴)- resultieren.

In der Formel I bedeutet A bevorzugt einen Cyclopropylring, so daß Verbindungen der Formel Ia resultieren: 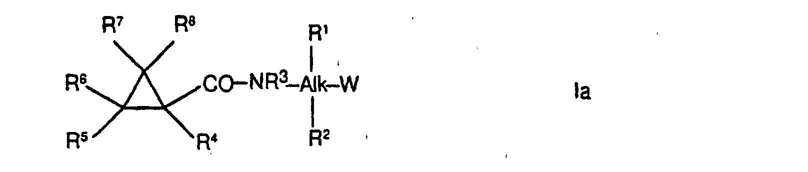 wobei die Substituenten R⁴- R⁸ folgende Bedeutungen haben:
- R⁴: Wasserstoff, Halogen, Cyano, C₁-C₆-Alkyl, C₂-C₆-Alkenyl, C₁-C₆-Alkoxy oder C₁-C₆-Alkylthio, wobei diese Reste partiell oder vollständig halogeniert sein und/oder eine oder zwei der folgenden Gruppen tragen können: C₁-C₄-Alkoxy, C₁-C₄-Halogenalkoxy, C₁-C₄-Alkylthio, C₁-C₄-Alkoxycarbonyl, C₃-C₆-Cycloalkyl, C₁-C₄-Alkoxycarbonylamino;
- R⁵: Wasserstoff, Halogen, Cyano, C₁-C₆-Alkyl, C₂-C₆-Alkenyl, C₁-C₆-Alkoxy oder C₁-C₆-Alkylthio, wobei diese Reste partiell oder vollständig halogeniert sein und/oder eine oder zwei der folgenden Gruppen tragen können: C₁-C₄-Alkoxy, C₁-C₄-Halogenalkoxy, C₁-C₄-Alkylthio, C₁-C₄-Alkoxycarbonyl, C₃-C₆-Cycloalkyl, C₁-C₄-Alkoxycarbonylamino;
- R⁶: Wasserstoff, Halogen oder C₁-C₆-Alkyl;
- R⁷: Wasserstoff, Halogen oder C₁-C₆-Alkyl;
- R⁸: Wasserstoff, Halogen oder C₁-C₆-Alkyl.

In der Formel Ia kommen für die Reste R⁴ - R⁸ insbesondere folgende Bedeutungen jeweils allein oder in Kombination miteinander in Frage:
a. Verbindungen der Formel Ia mit R⁴ Wasserstoff, Halogen, Cyano, C₁-C₆-Alkyl, C₁-C₄-Alkoxycarbonylamino; wie z.B. Methyl, Ethyl, Methoxycarbonylamino, Ethoxycarbonylamino oder Isopropyloxycarbonylamino.
b. Verbindungen der Formel Ia mit R⁵ Wasserstoff, C₁-C₆-Alkyl; wie z.B. Methyl oder Ethyl.
c. Verbindungen der Formel Ia mit R⁶ Wasserstoff, C₁-C₆-Alkyl; wie z.B. Methyl oder Ethyl; vorzugsweise Wasserstoff.
d. Verbindungen der Formale Ia mit R⁷ Wasserstoff, Chlor oder C₁-C₆-Alkyl; wie z.B. Methyl.
e. Verbindungen der Formel Ia mit R⁸ Wasserstoff, Chlor oder C₁-C₆-Alkyl; wie z.B. Methyl.

Der Cyclopropylring kann bezüglich R⁴- R⁸ ein- bis fünffach substituiert sein.

Monosubstituierte Cyclopropyl-Derivate der Formel Ia sind solche Verbindungen, bei denen insgesamt vier der Reste R⁴-R⁸ Wasserstoff bedeuten. Beispielsweise kommen solche Verbindungen in Frage, bei denen R⁴ Alkyl, Halogen, Cyano oder Alkoxycarbonylamino bedeutet.

Disubstituierte Cyclopropyl-Derivate der Formel Ia sind solche Verbindungen, bei denen insgesamt drei der Reste R⁴-R⁸ Wasserstoff bedeuten. Beispielsweise kommen solche Verbindungen in Frage, bei denen R⁴ C₁-C₆-Alkyl, Halogen oder Cyano und R⁵ C₁-C₆-Alkyl bedeutet. Bevorzugte disubstituierte Cyclopropyl-Derivate sind ferner solche Verbindungen, in denen R⁷ und R⁸ Halogen oder C₁-C₆-Alkyl bedeuten.

Trisubstituierte Cyclopropyl-Derivate der Formel Ia sind solche Verbindungen, bei denen insgesamt zwei der Reste R⁴-R⁸ Wasserstoff bedeuten. Beispielsweise kommen Verbindungen mit folgender Bedeutung in Frage: R⁴: C₁-C₆-Alkyl, Halogen oder Cyano; R⁸: Halogen oder C₁-C₆-Alkyl; R⁸: Halogen oder C₁-C₆-Alkyl.

Tetrasubstituierte Cyclopropyl-Derivate der Formel Ia sind Verbindungen, bei denen einer der Reste R⁴-R⁸ Wasserstoff bedeutet. Beispielsweise kommen Verbindungen in Frage, bei denen R⁵ oder R⁶ Wasserstoff bedeutet. Die übrigen Substituenten haben beispielsweise die folgenden Bedeutungen: R⁴: C₁-C₆-Alkyl, Halogen oder Cyano; R⁶: C₁-C₆-Alkyl; R⁷: Halogen oder C₁-C₆-Alkyl; R⁸: Halogen oder C₁-C₆-Alkyl.

Pentasubstituierte Cyclopropyl-Derivate der Formel Ia sind Verbindungen, bei denen keiner der Reste R⁴-R⁸ Wasserstoff bedeutet. Beispielsweise kommen solche Verbindungen in Frage, bei denen R⁴ C₁-C₆-Alkyl, Halogen oder Cyano und R⁵-R⁸ C₁-C₆-Alkyl bedeuten.

Unabhängig von dem zuvor genannten Substitutionsmuster kommen bevorzugt solche Verbindungen der Formel Ia in Frage, in denen R⁷ und R⁸ gleich sind und Halogen, insbesondere Chlor, oder C₁-C₆-Alkyl, insbesondere Methyl, bedeuten.

Für den Fall, daß die Alkylenkette Alk ein chirales Kohlenstoffatom enthält, weist dieses vorzugsweise die R-Konfiguration auf.

Sofern die Verbindungen der Formel Ia über ein oder mehrere Asymmetriezentren verfügen, kommen im Sinne der vorliegenden Erfindung sowohl die stereoisomeren Gemische als auch die entsprechenden Enantiomere oder Diastereomere in Frage. Bezüglich der stereochemischen Anordnung der Substituenten R⁴ und R⁵ kommen bevorzugt die Verbindungen der Formel Ib in Frage:

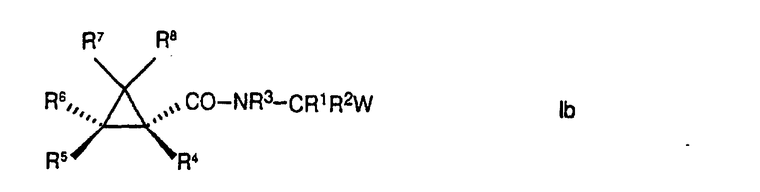

Insbesondere sind im Hinblick auf ihre Verwendung die in den anschließenden Tabellen 1 und 2 zusammengestellten Verbindungen Ia bevorzugt.

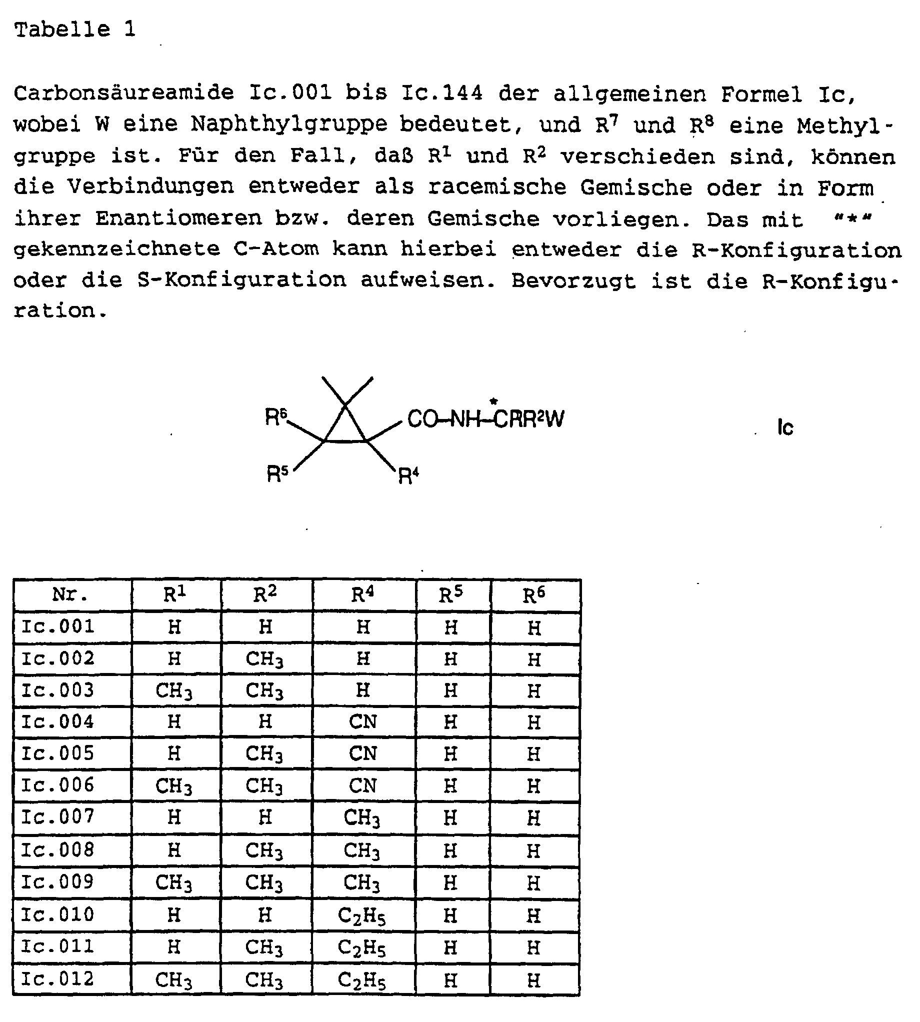

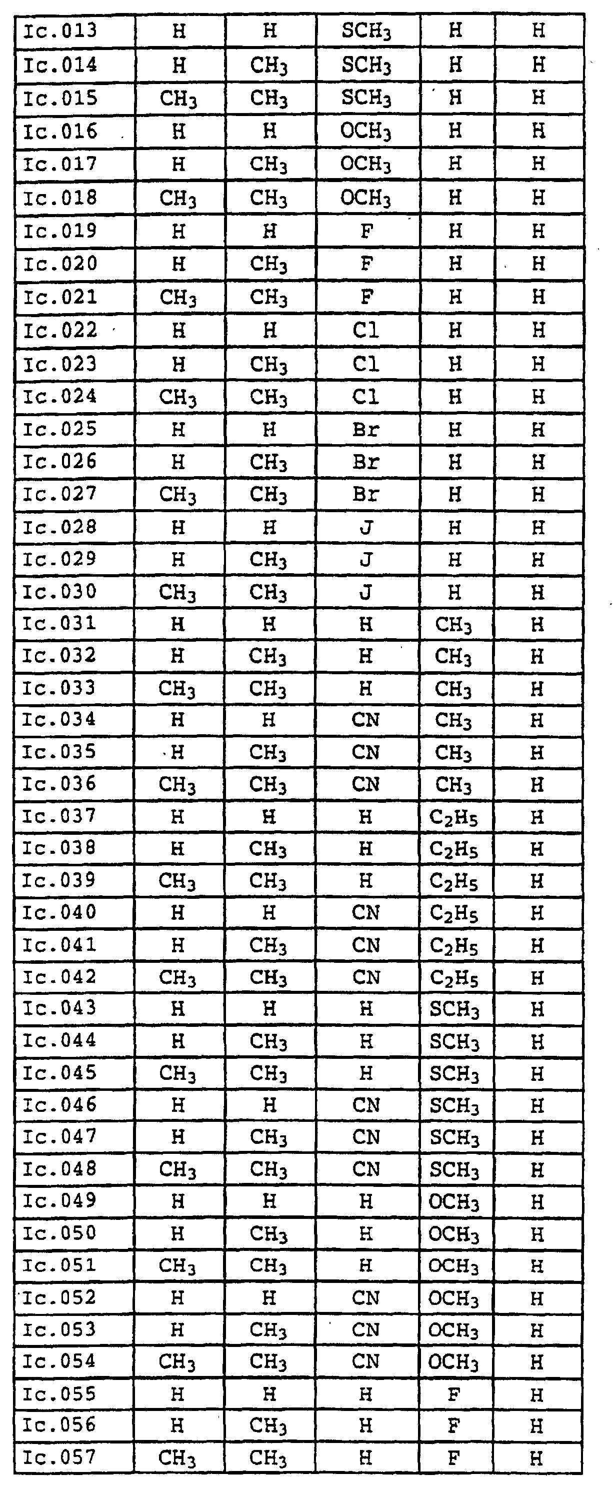

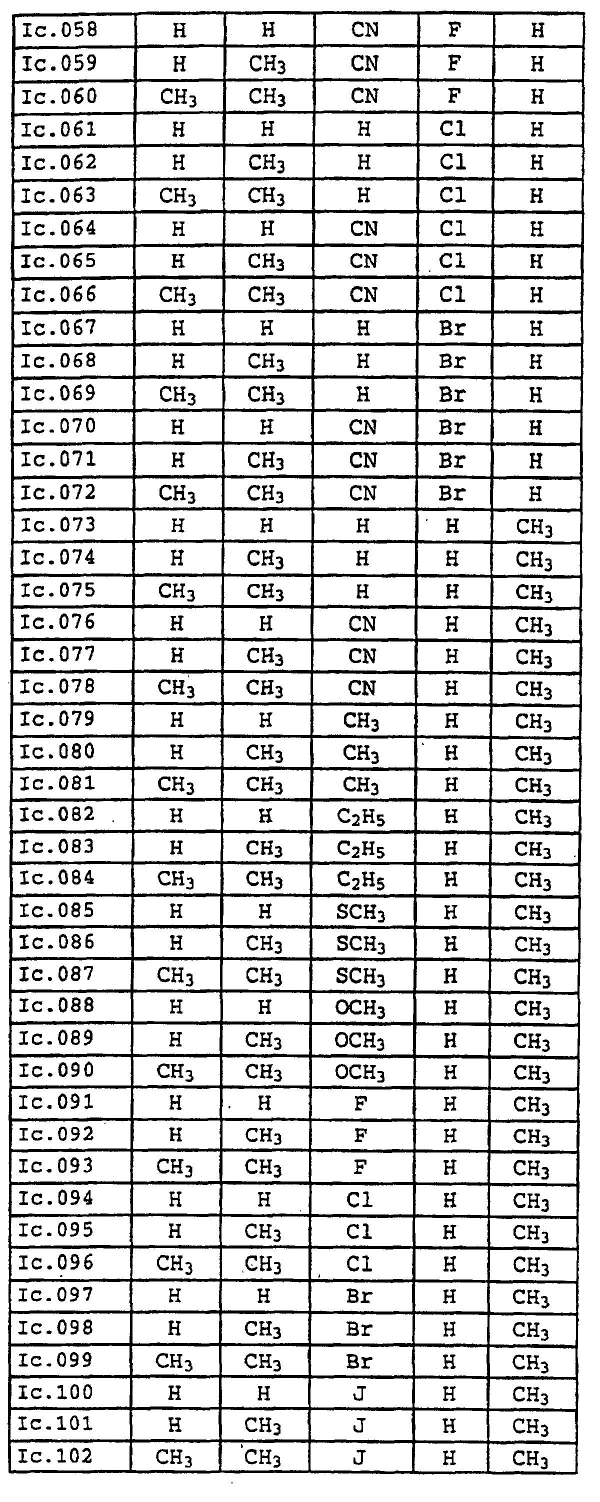

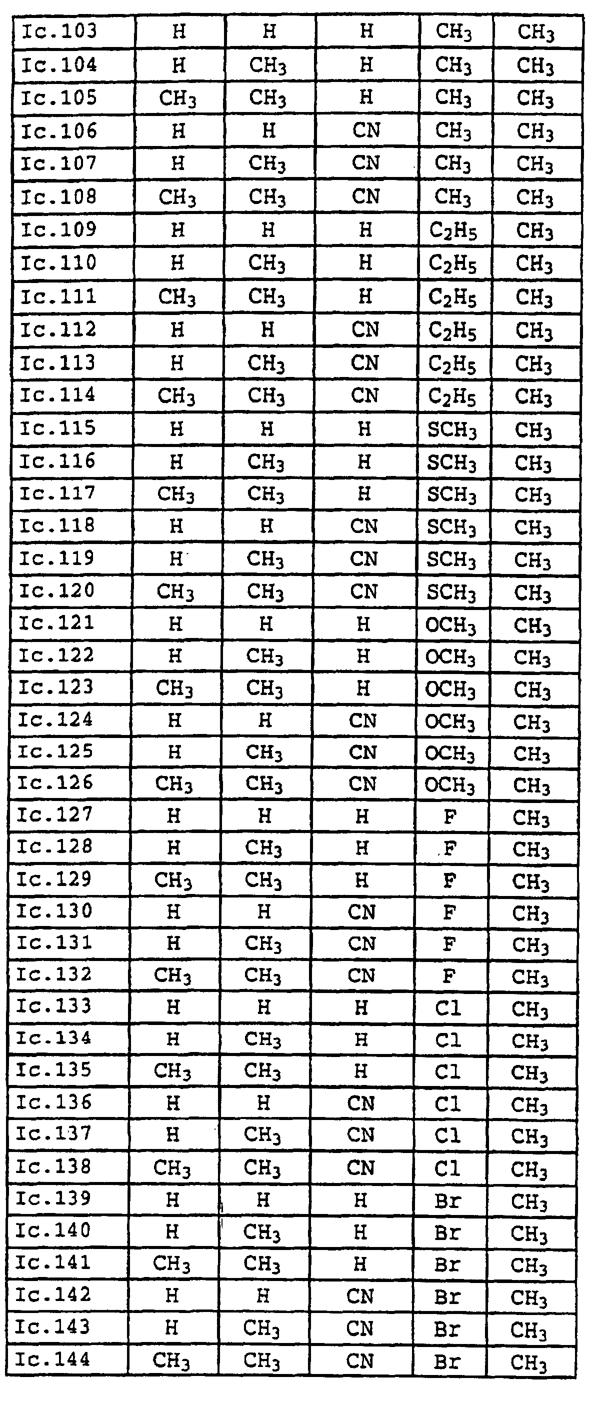

### Tabelle 2

Carbonsäureamide Id.001 bis Id.144 der allgemeinen Formel Id, in welcher die Bedeutungen der Kombinationen aus R¹-R⁶ durch die Zeilen der Tabelle 1 gegeben sind, W Naphthyl und R⁷ und R⁸ Chlor bedeuten.

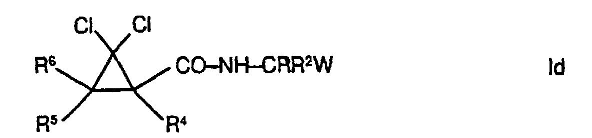

Nach einem erfindungsgemäß bevorzugten Verfahren erhält man die Carbonsäureamide I 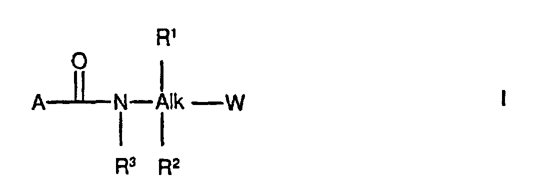 durch Umsetzung der Carbonsäurederivate II,

A-CO-X II

mit Aminen der Formel III

H₂N-Alk(R¹) (R²)-W III

wobei X eine leicht abspaltbare Gruppe darstellt.

Die Amidbildung erfolgt nach den literaturbekannten Verfahren. Dabei werden die freien Carbonsäuren der Formel II', wobei X für Hydroxy steht, in der Regel zuvor in eine aktiviertes Carbonsäurederivat II, wobei X z.B. für Chlor steht, übergeführt.

Die Aktivierung der Carbonsäuren II' kann vorzugsweise auch in situ erfolgen durch direkten Einsatz der Carbonsäuren II' unter Zusatz von z.B. Dicyclohexylcarbodiimid, Chlorameisensäureethylester, Cyanphosphonsäurediethylester, Triphenylphosphin/ Azodicarbonsäureester, 2-Pyridindisulfid/Triphenylphosphin, Carbonyldiimidazol, Thionylchlorid, Phosphortrichlorid, Phosphorpentachlorid, etc. Im allgemeinen erfolgt der Zusatz z.B. der Carbodiimide bezüglich der Carbonsäuren II' in äquimolaren Mengen.

Die Aktivierung der Carbonsäuren via Acylcyaniden geschieht beispielsweise durch Umsetzung der Carbonsäuren II' mit Cyanphosphonsäurediethylester, bevorzugt in einem inerten Lösungsmittel wie Tetrahydrofuran, Toluol oder Dichlormethan (vgl. Tetrahedron Lett. 18 (1973) 1595-8).

Die Aktivierung über Anhydride erfolgt beispielsweise durch Umsetzung der Carbonsäuren II' mit Kohlensäurechloriden wie Chlorameisensäureethylester im allgemeinen in Gegenwart von Basen und gegebenenfalls in einem inerten Lösungsmittel wie Toluol oder Tetrahydrofuran (vgl. "Houben-Weyl", 4. Aufl. (1974),15/1 Seite 28-32).

Die Amidbildung wird vorzugsweise in Gegenwart von Basen wie tertiären Aminen, z.B. Triethylamin oder Dimethylcyclohexylamin, Alkalimetallcarbonate, Alkalimetallhydroxide, Pyridin etc. durchgeführt. Die Reaktanden und die Hilfsbase werden zweckmäßigerweise in äquimolaren Mengen eingesetzt. Ein geringer Überschuß der Hilfsbase von 0,1 - 0,5 Äquivalenten kann unter Umständen hilfreich sein.

Als Lösungsmittel kommen aliphatische Kohlenwasserstoffe wie Hexan und Ligroin, aromatische Kohlenwasserstoffe wie Toluol und Xylol, chlorierte Kohlenwasserstoffe wie Methylenchlorid und 1,2-Dichlorethan, Ether wie Methyl-tert-butylether und Tetrahydrofuran, polare aprotische Lösungsmittel, wie Acetonitril und Dimethylformamid oder Ester wie Essigsäureethylester oder Gemische hiervon in Frage.

Das Molverhältnis von Carbonsäurederivate II zu Amin III beträgt im allgemeinen 0.8 bis 1,5 und vorzugsweise 0,9 bis 1,1.

Nach vollständiger Umsetzung wird wie üblich aufgearbeitet, z.B. durch Eintrag der Reaktionsmischung in Wasser und anschließende Extraktion des Amids mit einem organischen Lösungsmittel.

Die Carbonsäuren der Formel II sind bekannt oder können nach literaturbekannten Analogieverfahren hergestellt werden. Für den Fall, daß A einen Cyclopropylrest darstellt, können die Carbonsäuren nach dem folgenden Schema hergestellt werden:

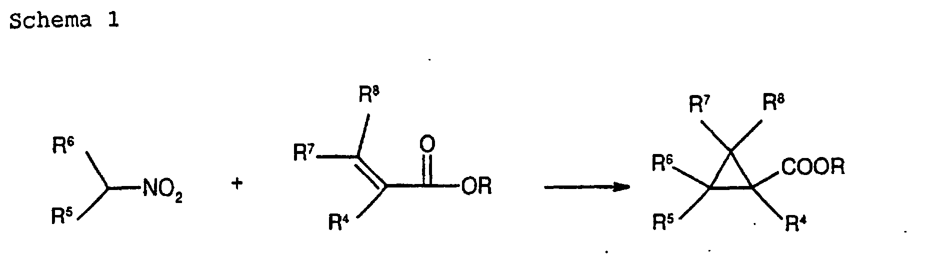

Die Amine der Formel III sind bekannt oder können nach literaturbekannten Verfahren erhalten werden (vgl. Organikum (1993) Barth Verlagsgesellschaft mbH Leipzig, S. 509 ff; "Houben- Weyl", Band 15/1, S. 648 - 665); Indian J. Chem. 10 (1972) 366).

Aus Racematen der Amine III kann das R-Isomere in an sich bekannter Weise, etwa durch fraktionierte Kristallisation mit optisch aktiver Weinsäure oder vorzugsweise mittels enzymkatalysierter Veresterung und anschließender Verseifung separiert werden (vgl. z.B. WO 95/08636).
Die Kondensation wird üblicherweise in einem mit Wasser nicht mischbaren Lösungsmittel wie Hexan, Toluol oder Xylol unter Auskreisen des bei der Reaktion entstehenden Wassers durchgeführt. Dazu wird das Reaktionsgemisch mehrere Stunden unter Rückfluß zum Sieden erhitzt.

Als Katalysatoren dienen Basen wie z.B. Piperidin, Pyridin, Ammoniak oder β-Alanin in Gegenwart einer Säure wie beispielsweise Eisessig.

X steht für einen nukleophil austauschbaren Rest wie Hydroxy, C₁-C₄-Alkoxy, Halogen z. B. Brom oder Chlor, Hetaryl, z.B. Imidazolyl oder Pyridyl, Carboxylat, z.B. Acetat oder Trifluoracetat etc.

Insbesondere bevorzugt sind Carbonsäurederivate der Formel II, in der A für gegebenenfalls substituiertes Cyclopropyl steht.

Die Verbindungen I zeichnen sich durch eine hervorragende Wirksamkeit gegen ein breites Spektrum von pflanzenpathogenen Pilzen, insbesondere aus der Klasse der Ascomyceten, Deuteromyceten, Phycomyceten und Basidiomyceten, aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an *Äpfeln,* Uncinula necator an Reben, *Puccinia*-Arten an Getreide, *Rhizoctonia*-Arten an Bau*mwolle*, *Reis und Rasen,* Ustilago-*Arten an Getreide und Zuckerrohr,* Venturia inaequalis *(Schorf) an Äpfeln,* Helminthosporium*-Arten* an Ge*treide,* Septoria nodorum an *Weizen,* Botrytis cinerea *(Grauschimmel) an Erdbeeren, Gemüse, Zierpflanzen und Reben,* Cercospora arachidicola *an Erdnüssen,* Pseudocercosporella herpotrichoides *an Weizen, Gerste,* Pyricularia oryzae *an Reis,* Phytophthora infestans *an Kartoffeln und Tomaten,* Fusarium- *und* Verticillium-*Arten an verschiedenen Pflanzen,* Plasmopara viticola *an Reben,* Pseudoperonospera*-Arten an Hopfen und Gurken,* Alternaria-*Arten an Gemüse und Obst, sowie* Mycosphaerella-*Arten in Bananen.*

Die Verbindungen I eignen sich außerdem zur Bekämpfung von Schadpilzen im Materialschutz (z.B. Holz, Papier, Dispersionen für den Ansprich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Kubikmeter behandelten Materials.

Die erfindungsgemäßen Mittel können in der Anwendungsform als Fungizide auch zusammen mit anderen Wirkstoffen vorliegen, wie z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-diisopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-ylethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
Strobilurine wie Methyl-E-methoxyimino-[α-(o-tolyloxy)-o-tolyl]acetat, Methyl-E-2-{2-[6-(2-cyanophenoxy)-pyrimidin-4-yloxy]-phenyl}-3-methoxyacrylat, Methyl-E-methoxyimino-[a-(2-phenoxyphenyl)]-acetamid, Methyl-E-methoxyimino-[α-(2,5-dimethylphenoxy)-o-tolyl]-acetamid,
Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
Phenylpyrrole wie 4-(2,2-Difluor-1,3-benzodioxol-4-yl)-pyrrol-3-carbonitril,
Zimtsäureamide wie 3-(4-Chlorphenyl)-3-(3,4-dimethoxyphenyl)-acrylsäuremorpholid,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat. DL-N-(2,6-Dimethyl-phenyl)-N-(2'-methoxyacetyl)-alanin-methyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

### Beispiel 1

### 1 Cyano-N-[(1R)-1-napht-2-yl-ethyl]-2,2,3,3-tetramethylcyclopropan-carboxamid

a) 2-Cyano-3-methyl-but-2-en-säuremethylester
   87 g (1,5 mol) Aceton wurden in 100 ml Benzol vorgelegt. Nach Zugabe von 131 g (1,33 mol) Cyanessigsäuremethylester, 16 g (0,28 mol) Essigsäure und 9,7 g (0,13 mol) Ammoniumacetat wurde 10 Stunden am Wasserauskreiser zum Rückfluß erhitzt. Zur Aufarbeitung wurde zweimal mit 1/4-konzentrierter Salzsäure bzw. Wasser gewaschen. Nach dem Trocknen mit Magnesiumsulfat und Entfernen des Lösungsmittels wurde das so erhaltene Rohprodukt destilliert. Es konnten 108 g (55 % Ausbeute) 2-Cyano-3-methyl-but-2-en-säuremethylester isoliert werden (Sdp. 95°C/5,2 mbar).
b) 1-Cyano-2,2,3,3-tetramethylcyclopropan-1-carbonsäuremethylester
   Die Synthese der Titelverbindung gelang in Anlehnung an J.Org.Chem. 1985, 50, 2807-2809: Es wurden 5,6 g (50 mmol) Kalium-tert-butanolat in 15 ml Dimethylsulfoxid vorgelegt und 4,4 g (50 mmol) Nitropropan, gelöst in 190 ml Dimethylsulfoxid, langsam zugetropft. Nach dem Zugeben von 5 g (36 mmol) 2-Cyano-3-methyl-but-2-en-säure-methylester, gelöst in 10 ml Dimethylsulfoxid, wurde bei Raumtemperatur 40 Stunden gerührt. Die Reaktion wurde durch die langsame Zugabe von Wasser abgebrochen. Es wurde mit Methyl-tertiär-Butylether dreimal extrahiert. Das mit Magnesiumsulfat getrocknete und vom Ether befreite Rohprodukt (9 g) wurde ohne weitere Aufreinigung in der Folgereaktion eingesetzt.
c) 1-Cyano-2,2,3,3-tetramethylcyclopropan-1-carbonsäure
   Die Hydrolyse des als Rohprodukt eingesetzten (9 g) 1-Cyano-2,2,3,3-tetramethylcyclopropyl-1-carbonsäuremethylesters gelang durch 4-stündiges Refluxieren in einer Mischung aus gleichen Teilen Methanol, Tetrahydrofuran und 2 n Natronlauge (je 70 ml). Zur Aufarbeitung gab man das gleiche Volumen 2 n Natronlauge hinzu und extrahierte mehrmals mit Ether. Nach Ansäuern der wässrigen Phase mit Salzsäure ließ sich durch Extraktion mit Methylenchlorid, Trocknen mit Magnesiumsulfat und Entfernen des Lösungsmittels 1-Cyano-2,2,3,3-tetramethylcyclopropyl-1-carbonsäure als weißer Feststoff isolieren (5,5 g; 66 % Ausbeute über 2 Stufen).
d) 1-Cyano-N[(1R)-1-naphth-2-yl-ethyl]-2,2,3,3-tetramethylcyclopropancarboxamid
   Zu einer Lösung von 1,0 g (6 mmol) 1-Cyano-2,2,3,3-tetramethylcyclopropan-1-carbonsäure (vgl. hierzu Org. Prep. Proced. Int. Bd. 5, (1973), S. 25-29) und 1,03 g (6 mmol) (1 R)-1-Naphth-2-yl-ethylamin in 50 ml Dichlormethan wurden 0,61 g (6 mmol) Triethylamin gegeben. Sodann wurden 0,94 g 93 %-iger (6 mmol) Cyanphosphonsäurediethylester bei 10°C zugetropft, und es wurde 12 Stunden bei Raumtemperatur gerührt. Nach Einengen am Rotationsverdampfer wurde die Reaktionslösung chromatographisch an Kieselgel gereinigt (Laufmittel: Methyl-tert.-Butylether : Hexan = 1 : 9, dann 3 : 7). Es konnten 1,9 g (99% Ausbeute) der Titelverbindung als weißer Feststoff mit einem Schmelzpunkt von 169-175°C isoliert werden.

### Beispiel 2

### (1R,3S/1S,3R)-2,2-Dichlor-N[(1R)-1-naphth-2-ylethyl]-1-ethyl-3-methyl-cyclopropancarboxamid

a) 2,2-Dichlor-l-ethyl-3-methylcyclopropan-1-carbonsäureethylester
   38 g (0,27 mol) 2-Ethyl-but-2-ensäureethylester, 2,2 g (0,01 mol) Benzyltriethylammoniumchlorid und 57 ml 50%-ige Natronlauge wurden in 180 ml Dichlormethan vorgelegt. Bei 35°C - 40°C wurden innnerhalb einer Stunde 95 ml Chloroform zugetropft. Nach 5 Stunden Reaktionszeit bei 40°C wurde noch weitere 14 Stunden bei Raumtemperatur gerührt. Zur Aufarbeitung wurde die Reaktionslösung auf 1,5 1 Wasser gekippt. Nach Abtrennen der organischen Phase wurde die wässrige Phase mit Dichlormethan extrahiert. Die vereinten organischen Phasen wurden mit Wasser gewaschen und nach dem Trocknen mit Magnesiumsulfat eingeengt. Der Rückstand (47 g) wurde einer Vakuumdestillation unterworfen. Die Titelverbindung konnte als farblose Flüssigkeit isoliert werden (25,7 g, 43 % Ausbeute, Siedepunkt 71°C bei 2 mbar).
b) 2,2-Dichlor-1-ethyl-3-methylcyclopropan-1-carbonsäure Die Verseifung des Esters gelang analog Beispiel 1d). Das Produkt konnte als gelblich-weißes Harz isoliert werden (19,7 g; 91 % Ausbeute).
c) 2,2-Dichlor-1-ethyl-3-methylcyclopropan-1-carbonsäurechlorid Zur Generierung des Säurechlorids wurden 19,7 g (0,1 mol) 2,2-Dichlor-1-ethyl-3-methylcyclopropan-1-carbonsäure in Toluol gelöst und 36,9 g (0,31 mol) Thionylchlorid zugegeben. Nach einer Reaktionszeit von 5 Stunden am Rückfluß wurde weitere 14 Stunden bei Raumtemperatur gerührt. Die Reaktionslösung wurde am Wasserstrahlvakuum eingeengt. Als Rohprodukt wurden 22 g einer braunen Flüssigkeit isoliert. Vor der weiteren Verwendung erfolgte keine Aufreinigung.
d) (1R,3S/1S,3R)-2,2-Dichlor-N[(1R)-1-naphth-2-ylethyl]-1-ethyl-3-methylcyclopropancarboxamid 0,62 g (2,9 mmol) 2,2-Dichlor-1-ethyl-3-methylcyclopropan-1-carbonsäurechlorid wurden in 20 ml abs. Dichlormethan vorgelegt. Nach Zugabe von 0,5 g (2,9 mmol) (1R)-1-Naphth-2-yl-ethylamin und 0,3 g (2,9 mmol) Triethylamin wurde bei Raumtemperatur 16 Stunden gerührt. Zur Aufarbeitung wurde die Reaktionslösung mit dem gleichen Volumen Dichlormethan verdünnt und mit 2 n Salzsäure, 2 n Natronlauge sowie Wasser gewaschen. Nach dem Trocknen der organischen Phase mit Magnesiumsulfat und Entfernen des Lösungsmittels konnte die Titelverbindung als Feststoff isoliert werden (0,9 g; 81 % Ausbeute). Das Diastereomerengemisch hat einen Schmelzpunkt von 120 - 135°C.

### Beispiel 3

### (1S,3R/1R,3S)-2,2-Dichlor-1-ethyl-3-methyl-cyclopropancarbonsäure [(1R/1S)-1-methyl-1-naphth-2-yl-propyl]amid

### Analog zu Beispiel 1d), jedoch mit doppelter Triethylaminmenge, gelang die Umsetzung von 0,94 g (4 mmol)

2,2-Dichlor-1-ethyl-3-methylcyclopropan-1-carbonsäurechlorid mit 0,86 g (4 mmol) 1-Methyl-1-naphth-2-ylpropylaminhydrochlorid zur Titelverbindung. Nach säulenchromatographischer Reinigung an Kieselgel (Laufmittel: Cyclohexan-Essigsäureethylester-Gradient) wurde das harzige Produkt als Diastereomerengemisch isoliert (1,1 g; 73 % Ausbeute).
Physikalische Daten (NMR in CDCl₃, Angaben in ppm) = ¹H-NMR: 0,8; 1,1; 1,2; 1,3; 1,6; 1,9; 2,2.

### Beispiel 4

### (1S,3R/1R,3S)-2,2-Dichlor-1-ethyl-3-methyl-cyclopropancarbonsäure-[1R/1S)-1-methyl-1-naphth-1-yl-propyl]amid

Analog zu Beispiel 1d) gelang die Umsetzung von 0,25 g (1,1 mmol) 2,2-Dichlor-1-ethyl-3-methylcyclopropan-1-carbonsäurechlorid mit 0,23 g (1,1 mmol) 1-Methyl-1-naphth-1-yl-propylaminhydrochlorid. Die Titelverbindung konnte nach säulenchromatographischer Reinigung an Kieselgel (Laufmittel: Cyclohexan-Essigsäureethylester-Gradient) als harziges Diastereomerengemisch isoliert werden (0,15 g; 38 % Ausbeute). ¹H-NMR: 0,8; 1,1 - 1,3; 1,6; 2,0; 2,5 - 2,6; 6,2; 7,4; 7,6; 7,8 - 8,0; 8,6.

### Beispiel 5

1-Methylcyclopropancarbonsäure [(1R)-1-naphth-2-yl-ethyl]-amid Analog zu Beispiel 1d) gelang die Umsetzung von 1,5 g (15 mmol) 1-Methyl-cyclopropan-1-carbonsäure mit 2,6 g (15 mmol) (1R)-1-Naphth-2-yl-ethylamin zur Titelverbindung. Nach Säulenchromatographischer Reinigung an Kieselgel (Laufmittel:Cyclohexan/Essigsäureethylester-Gradient) wurde das Produkt als weißer Feststoff isoliert (2,9 g; 76 % Ausbeute, Schmelzpunkt 91°C).

### Beispiel 6

### 1-Cyano-2,2,3-trimethylcyclopropancarbonsäure[(1R)-1-naphth-2-ylethyl]-amid

a) 2-Cyano-but-2-en-säuremethylester
   In Analogie zu Beispiel 1a) wurde Acetaldehyd in einer Knoevenagel-Reaktion mit Cyanessigsäuremethylester umgesetzt zu 2-Cyano-but-2-ensäuremethylester.
b) 1-cyano-2,2,3-trimethylcyclopropan-1-carbonsäuremethylester
   In Analogie zu Beispiel 1b) gelang die Umsetzung von 2-Cyanobut-2-ensäuremethylester zu 1-Cyano-2,2,3-trimethylcyclopropan-1-carbonsäuremethylester.
c) 1-Cyano-2,2,3-trimethylcyclopropan-1-carbonsäure
   Die Verseifung von 1-Cyan-2,2,3-trimethylcyclopropan-1-carbonsäuremethylester zur 1-Cyan-2,2,3-trimethylcyclopropan-1-carbonsäure gelang in Analogie zu Beispiel 1c).
d) 1-Cyano-2,2,3-trimethylcyclopropancarbonsäure[(1R)-1-naphth-2-yl-ethyl]-amid
   Die Amidbildung aus 1-Cyano-2,2,3-trimethylcyclopropan-1-carbonsäure und (1R)-1-Naphth-2-yl-ethylamin gelang in Analogie zu Beispiel 1d). Die Titelverbindung wurde als gelbes Harz isoliert.
   ¹H-NMR: 1,2 - 1,3; 1,4; 1,6; 2,1; 5,3; 6,5; 7,4; 7,8

### Beispiel 7

### [1-(1R)-1-Naphth-2-yl-ethylcarbamoyl)-cyclopropyl]-carbaminsäureisopropylester

a) 1-Isopropoxycarbonylamino-cyclopropancarbonsäure
   0,2 g (1,98 mmol) 1-Aminocyclopropan-1-carbonsäure wurden in 10 ml 12%-iger Natronlauge vorgelegt. Nach Zugabe von 0,24 g (1,98 mmol) Isopropylchlorformiat wurde durch tropfenweises Hinzufügen von 12%-iger Natronlauge ein pH-Wert von 9 eingestellt. Nach 14-stündigem Rühren bei Raumtemperatur wurde zur Aufarbeitung 2X mit Methyl-tert.-Butylether extrahiert. Anschließlich wurde mit Phosphorsäure ein pH-Wert von 1-2 eingestellt. Nach dreimaliger Extraktion mit Dichlormethan, Trocknen mit Magnesiumsulfat und Entfernen des Lösungsmittels konnte die Titelverbindung als weißer Feststoff isoliert werden (0,2 g; 54 % Ausbeute).
b) [1-(1R)-1-Naphth-2-yl-ethylcarbamoyl)-cyclopropyl]carbaminsäureisopropylester
   Zur Darstellung des Amids wurden 0,1 g (0,53 mmol) 1-Isopropoxy-cabonylaminocyclopropancarbonsäure in 30 ml Dichlormethan vorgelegt. Nach Kühlen auf 10°C wurden 0,5 g (0,53 mmol) Triethylamin und 0,57 g (0,53 mmol) Ethylchlorformiat zugegeben. Nach 2 Stunden Rühren wurden weitere 0,5 g (0,53 mmol) Triethylamin sowie 0,9 g (0,53 mmol) (1R)-1-Naphth-2-yl-ethylamin zugetropft. Nach 16 Stunden Rühren bei Raumtemperatur wurde zur Aufarbeitung einmal mit 5%-iger Natronlauge, zweimal mit 2n Salzsäure und einmal mit Wasser gewaschen. Nach dem Trocknen der organischen Phase mit Magnesiumsulfat und Entfernen des Lösungsmittels wurde der Rückstand einer säulenchromatographischen Reinigung an Silicagel unterworfen (Laufmittel: Cyclohexan: Methyl-tert.-Butylether = 9:1, 6:4, 1:1). Die Titelverbindung wurde als weiß-gelbliches Harz isoliert (0,14 g; 75 % Ausbeute).
   ¹H-NMR: 0,8 - 1,0; 1,2; 1,4; 1,6; 4,9 - 5,1; 5,3; 6,8; 7,5; 7,8.

### Beispiel 8

### 3-(2,2)-Dichlorvinyl)-2,2-dimethylcyclopropancarbonsäure [(1R)-1-napht-2-yl-ethyl-amid]

Die Ausgangsverbindung wurde erhalten gemäß J. Org. Chem. 1988, 53 (16), 3843 - 3845. 0,47 g (2,24 mmol) 3-(2,2-Dichlorvinyl)-2,2-dimethylcyclopropansäure wurden in 50 ml abs. Methylenchlorid gelöst und mit 0,22 g (2,24 mmol) Triethylamin sowie 0,38 g (2,24 mmol) (1R)-1-Napht-2-yl-ethylamin versetzt. Der Reaktionskolben wurde auf 0°C abgekühlt, dann erfolgte die Zugabe von 0,33 g (2,02 mmol) 93 %-igem Cyanessigsäureethylester.

Nach Beendigung der Zugabe wurde das Eisbad entfernt, und es wurde über Nacht gerührt. Zur Aufarbeitung wurde die Reaktionslösung hintereinander mit 2n Natronlauge, Wasser, 2n Salzsäure, Wasser, 2n Natronlauge und schließlich noch einmal mit Wasser gewaschen. Nach dem Trocknen der organischen Phase mit Magnesiumsulfat und Entfernen des Lösungsmittels wurde das Produkt durch säulenchromatographische Trennung an Kieselgel (Laufmittel: Cyclohexan-Essigsäureethylester 9,5 : 0,5; dann 7 : 3) als gelbes Harz isoliert. Entsprechend dem Einsatz der diastereomeren Ausgangsverbindung wurde auch die Titelverbindung 3-(2,2-Dichlorvinyl)-2,2-dimethylcyclopropancarbonsäure [(1R)-1-naphth-2-ylethyl]amid als Diastereomerengemisch erhalten (0,5 g; 62 % Ausbeute).

Physikalische Daten (¹H-NMR, CDCl₃, in ppm):
1,1 - 1,3; 1,4; 1,6; 1,9; 2,3; 5,3; 5,6; 5,8; 6,4; 7,4; 7,7 - 7,9.

### Beispiel 9

### 3-(2-Chlor-3,3,3-trifluorpropenyl)-2,2-dimethylcyclopropancarbonsäure[(1R)-1-naphth-2-yl-ethyl]-amid, (E)-Isomer

Die Ausgangsverbindung wurde als Diastereomerengemisch erhalten gemäß Patent JP 62/116531.

0,54 g (2,24 mmol) (E)-3-(2-Chlor-3,3,3-trifluorpropenyl)-2,2-dimethylcyclopropancarbonsäure wurden in 50 ml abs. Methylenchlorid gelöst und mit 0,22 g (2,24 mmol) Triethylamin sowie 0,38 g (2,24 mmol) (1R)-1-Naphth-2-yl-ethylamin versetzt. Der Reaktionskolben wurde auf 0°C abgekühlt, dann erfolgte die Zugabe von 0,33 g (2,02 mmol) 93 %-igem Cyanessigsäureethylester.

Nach Beendigung der Zugabe wurde das Eisbad entfernt, und es wurde über Nacht gerührt. Zur Aufarbeitung wurde die Reaktionslösung mit 2n Natronlauge, dann Wasser, 2n Salzsäure, Wasser, 2n Natronlauge und schließlich noch einmal mit Wasser gewaschen. Nach dem Trocknen der organischen Phase mit Magnesiumsulfat und Entfernen des Lösungsmittels wurde das Produkt durch säulenchromatographische Trennung an Kieselgel (Laufmittel: Cyclohexan/Essigsäureethylester 9,5 : 0,5; dann 7 : 3) isoliert. Es wurden 0,7 g (79 % Ausbeute) der diastereomeren Titelverbindung als gelbliche Kristalle (Schmelzpunkt 75°C - 77°C) erhalten.

### Beispiel 10

### 3-(2-Chlor-3,3,3-trifluorpropenyl)2,2-dimethylcyclopropancarbonsäure[(1R)-1-naphth-2-yl-ethyl]amid, (Z)-Isomer

Die Ausgangsverbindung wurde als Diastereomerengemisch erhalten gemäß Patent JP 62/116531.

0,54 g (2,24 mmol) (E)-3-(2-Chlor-3,3,3-trifluorpropenyl)-2,2-dimethylcyclopropancarbonsäure wurde in 50 ml abs. Methylenchlorid gelöst und mit 0,22 g (2,24 mmol) Triethylamin sowie 0,38 g (2,24 mmol) (1R)-1-Naphth-2-yl-ethylamin versetzt. Der Reaktionskolben wurde auf 0°C abgekühlt, dann erfolgte die Zugabe von 0,33 g (2,02 mmol) 93 %-igem Cyanessigsäureethylester. Nach Beendigung der Zugabe wurde das Eisbad entfernt, und es wurde über Nacht gerührt. Zur Aufarbeitung wurde die Reaktionslösung mit 2n Natronlauge, Wasser, 2n Salzsäure, Wasser, 2n Natronlauge und schließlich noch einmal mit Wasser gewaschen. Nach dem Trocknen der organischen Phase mit Magnesiumsulfat und Entfernen des Lösungsmittels wurde das Produkt durch säulenchromatographische Reinigung an Kieselgel (Laufmittel Cyclohexan:Essigsäureethylester = 9,5 : 0,5; dann 7 : 2) isoliert. Es wurden 0,6 g (68 % Ausbeute) der diastereomeren Titelverbindung als gelbes Harz erhalten.

Physikalische Daten: (¹H, CDCl₃, in ppm):
1,2 - 1,3; 1,4; 1,5 - 1,6; 2,5; 5,3; 5,9; 6,1; 7,4; 7,7 - 7,9.

### Beispiel 11

### Anwendungsbeispiele

Die fungizide Wirkung der Verbindungen der allgemeinen Formel I gegen Schadpilze ließ sich durch folgende Gewächshausversuche zeigen:

Die Wirkstoffe wurden als 20 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

### 1. Wirksamkeit gegen Pyricularia oryzae (protektiv)

Blätter von in Töpfen gewachsenen Reiskeimlingen (Sorte "Tai-Nong 67") wurden mit der wäßrigen Aufbereitung der Wirkstoffe (250 ppm-haltig) behandelt. Nach ca. 24 Stunden wurden die Pflanzen mit einer wäßrigen Sporensuspension von Pyricularia oryzae inokuliert. Die so behandelten Pflanzen wurden in Klimakammern bei 22 - 24°C und 95 bis 99% relativer Luftfeuchte für 6 Tage aufgestellt. Anschließend wurde das Ausmaß der Befallsentwicklung auf den Blättern visuell ermittelt.

In diesem Test zeigten die mit den erfindungsgemäßen Verbindungen behandelten Pflanzen einen geringen Befall und die unbehandelten Pflanzen 80 % Befall.

### 2. Systemische Wirksamkeit gegen Pyricularia oryzae

Vorgekeimter Reis (Sorte "Tai-Nong 67") wurde in einer Hydrokultur mit Hoagland-Lösung bis zum Zweiblattstadium kultiviert. Dann wurden die Wurzeln mit der wäßrigen Aufbereitung der Wirkstoffe (50 ppm-haltig) angegossen. Nach fünf Tagen weiterer Kultivierung im Gewächshaus wurden die Pflanzen mit einer wäßrigen Sporensuspension von *Pyricularia oryzae* inokuliert. Die so behandelten Pflanzen wurden in Klimakammern bei 22 - 24°C und 95 bis 99% relativer Luftfeuchte für 6 Tage aufgestellt. Anschließend wurde das Ausmaß der Befallsentwicklung auf den Blättern visuell ermittelt.

In diesem Test zeigten die mit den erfindungsgemäßen Verbindungen behandelten Pflanzen einen geringen Befall und die unbehandelten Pflanzen 80% Befall.

## Patentansprüche

1. Cycloalkylcarbonsäureamide der allgemeinen Formel Ia 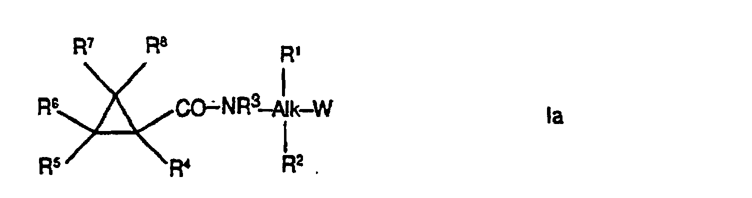 mit folgenden Bedeutungen:
Alk geradkettiges oder verzweigtes C₁-C₆-Alkylen;
R¹ C₁-C₆-Alkyl oder C₂-C₆-Alkenyl, wobei diese Reste partiell oder vollständig halogeniert sein und/oder eine oder zwei der folgenden Gruppen tragen können: C₁-C₄-Alkoxy, C₁-C₄-Halogenalkoxy, C₁-C₄-Alkylthio, C₁-C₄-Alkoxycarbonyl, C₃-C₆-Cycloalkyl und Phenyl, wobei das Phenyl partiell oder vollständig halogeniert sein und/oder einen bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy, C₁-C₄-Alkylthio, C₃-C₆-Cycloalkyl oder Heterocyclyl;
R², R³ Wasserstoff, C₁-C₆-Alkyl oder C₂-C₆-Alkenyl, wobei diese Reste partiell oder vollständig halogeniert sein können;
W kondensiertes bicyclisches Ringsystem mit jeweils sechs Ringatomen, wobei ein oder zwei C-Ringatome durch N-Atome ausgetauscht sein können, und wobei diese Ringsysteme eine bis drei der folgenden Gruppen tragen können: Nitro, Halogen, Cyano, C₁-C₄-Alkyl, C₁-C₄-Halogenalkyl, C₁-C₄-Alkoxy, C₁-C₄-Halogenalkoxy, C₁-C₄-Alkylthio, C₃-C₆-Cycloalkyl und C₁-C₄-Alkoxycarbonyl;
R⁴ Wasserstoff, Halogen, Cyano, C₁-C₆-Alkyl, C₂-C₆-Alkenyl, C₁-C₆-Alkoxy oder C₁-C₆-Alkylthio, wobei diese Reste partiell oder vollständig halogeniert sein und/oder eine oder zwei der folgenden Gruppen tragen können: C₁-C₄-Alkoxy, C₁-C₄-Halogenalkoxy, C₁-C₄-Alkylthio, C₁-C₄-Alkoxycarbonyl, C₃-C₆-Cycloalkyl, C₁-C₄-Alkoxycarbonylamino;
R⁵ Wasserstoff, Halogen, Cyano, C₁-C₆-Alkyl, C₂-C₆-Alkenyl, C₁-C₆-Alkoxy oder C₁-C₆-Alkylthio, wobei diese Reste partiell oder vollständig halogeniert sein und/oder eine oder zwei der folgenden Gruppen tragen können: C₁-C₄-Alkoxy, C₁-C₄-Halogenalkoxy, C₁-C₄-Alkylthio, C₁-C₄-Alkoxycarbonyl, C₃-C₆-Cycloalkyl, C₁-C₄-Alkoxycarbonylamino;
R⁶ Wasserstoff, Halogen oder C₁-C₆-Alkyl;
R⁷ Wasserstoff, Halogen oder C₁-C₆-Alkyl;
R⁸ Wasserstoff, Halogen oder C₁-C₆-Alkyl,
sowie deren landwirtschaftlich einsetzbaren Salze.

2. Cycloalkylcarbonsäureamide der allgemeinen Formel Ia gemäß Anspruch 1, wobei der Cyclopropylring einen bis fünf Substituenten R⁴ - R⁸ ausgewählt aus der Gruppe der Reste bestehend aus Halogen, Cyano und C₁-C₃-Alkyl tragen kann.

3. Cycloalkylcarbonsäureamide nach Anspruch 1, wobei R⁴ Halogen, Cyano, C₁-C₆-Alkyl, C₁-C₄-Alkoxycarbonylamino, C₁-C₄-Alkoxy oder C₁-C₄-Alkylthio bedeutet.

4. Cycloalkylcarbonsäureamide nach einem der Ansprüche 1 - 3, wobei R⁵ Wasserstoff oder C₁-C₆-Alkyl bedeutet.

5. Cycloalkylcarbonsäureamide nach einem der Ansprüche 1 - 4, wobei R⁶ Wasserstoff oder C₁-C₆-Alkyl bedeutet.

6. Cycloalkylcarbonsäureamide nach einem der Ansprüche 1 - 5, wobei R⁷ Wasserstoff, Chlor oder C₁-C₆-Alkyl bedeutet.

7. Cycloalkylcarbonsäureamide nach einem der Ansprüche 1 - 6, wobei R⁸ Wasserstoff, Chlor oder C₁-C₆-Alkyl bedeutet.

8. Cycloalkylcarbonsäureamide nach einem der Ansprüche 1 - 7, wobei R¹ C₁-C₆-Alkyl und R² Wasserstoff oder C₁-C₆-Alkyl bedeuten.

9. Cycloalkylcarbonsäureamide nach einen der Ansprüche 1 - 8, wobei Alk eine Methylengruppe bedeutet.

10. Mittel, enthaltend eine zur Bekämpfung von Schadpilzen wirksame Menge mindestens eines Cycloalkylcarbonsäureamids der Formel I gemäß einem der Ansprüche 1 - 9 und gegebenenfalls einen inerten flüssigen und/oder festen Trägerstoff sowie gegebenenfalls mindestens einen oberflächenaktiven Stoff.

11. Verfahren zur Bekämpfung von Schadpilzen, **dadurch gekennzeichnet, daß** man die Schadpilze, deren Lebensraum oder die von ihnen freizuhaltenden Pflanzen, Flächen, Materialien oder Räume mit einer wirksamen Menge eines Cycloalkylalkancarbonsäureamids der Formel I gemäß einem der Ansprüche 1 - 9 behandelt.

12. Verwendung eines Cycloalkylalkancarbonsäureamids der allgemeinen Formel I gemäß einem der Ansprüche 1 - 9 zur Bekämpfung von Schadpilzen.

## Claims

1. A cycloalkylcarboxamide of the formula Ia 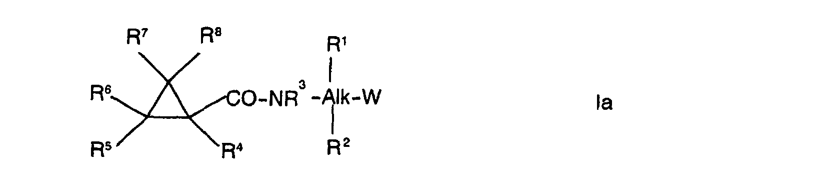 where:
Alk is straight-chain or branched C₁-C₆-alkylene;
R¹ is C₁-C₆-alkyl or C₂-C₆-alkenyl, where these radicals may be partially or fully halogenated and/or may carry one or two of the following groups: C₁-C₄-alkoxy, C₁-C₄-haloalkoxy, C₁-C₄-alkylthio, C₁-C₄-alkoxycarbonyl, C₃-C₆-cycloalkyl and phenyl, where the phenyl may be partially or fully halogenated and/or may carry one to three of the following radicals: nitro, cyano, C₁-C₄-alkyl, C₁-C₄-haloalkyl, C₁-C₄-alkoxy, C₁-C₄-haloalkoxy, C₁-C₄-alkylthio, C₃-C₆-cycloalkyl or heterocyclyl;
R², R³ are hydrogen, C₁-C₆-alkyl or C₂-C₆-alkenyl, where these radicals may be partially or fully halogenated;
W is a fused bicyclic ring system having in each case six ring atoms, where one or two carbon ring atoms may be replaced by nitrogen atoms, and where these ring systems may carry one to three of the following groups: nitro, halogen, cyano, C₁-C₄-alkyl, C₁-C₄-haloalkyl, C₁-C₄-alkoxy, C₁-C₄-haloalkoxy, C₁-C₄-alkylthio, C₃-C₆-cycloalkyl and C₁-C₄-alkoxycarbonyl;
R⁴ is hydrogen, halogen, cyano, C₁-C₆-alkyl, C₂-C₆-alkenyl, C₁-C₆-alkoxy or C₁-C₆-alkylthio, where these radicals may be partially or fully halogenated and/or may carry one or two of the following groups: C₁-C₄-alkoxy, C₁-C₄-haloalkoxy, C₁-C₄-alkylthio, C₁-C₄-alkoxycarbonyl, C₃-C₆-cycloalkyl, C₁-C₄-alkoxycarbonylamino;
R⁵ is hydrogen, halogen, cyano, C₁-C₆-alkyl, C₂-C₆-alkenyl, C₁-C₆-alkoxy or C₁-C₆-alkylthio, where these radicals may be partially or fully halogenated and/or may carry one or two of the following groups: C₁-C₄-alkoxy, C₁-C₄-haloalkoxy, C₁-C₄-alkylthio, C₁-C₄-alkoxycarbonyl, C₃-C₆-cycloalkyl, C₁-C₄-alkoxycarbonylamino;
R⁶ is hydrogen, halogen or C₁-C₆-alkyl;
R⁷ is hydrogen, halogen or C₁-C₆-alkyl;
R⁸ is hydrogen, halogen or C₁-C₆-alkyl,
and their agriculturally useful salts.

2. A cycloalkylcarboxamide of the formula Ia as claimed in claim 1, where the cyclopropyl ring may carry one to five substituents R⁴ - R⁸ selected from the group of the radicals consisting of halogen, cyano and C₁-C₃-alkyl.

3. A cycloalkylcarboxamide as claimed in claim 1, where R⁴ is halogen, cyano, C₁-C₆-alkyl, C₁-C₄-alkoxycarbonylamino, C₁-C₄-alkoxy or C₁-C₄-alkylthio.

4. A cycloalkylcarboxamide as claimed in any of claims 1 - 3, where R⁵ is hydrogen or C₁-C₆-alkyl.

5. A cycloalkylcarboxamide as claimed in any of claims 1 - 4, where R⁶ is hydrogen or C₁-C₆-alkyl.

6. A cycloalkylcarboxamide as claimed in any of claims 1 - 5, where R⁷ is hydrogen, chlorine or C₁-C₆-alkyl.

7. A cycloalkylcarboxamide as claimed in any of claims 1 - 6, where R⁸ is hydrogen, chlorine or C₁-C₆-alkyl.

8. A cycloalkylcarboxamide as claimed in any of claims 1 - 7, where R¹ is C₁-C₆-alkyl and R² is hydrogen or C₁-C₆-alkyl.

9. A cycloalkylcarboxamide as claimed in any of claims 1 - 8, where Alk is a methylene group.

10. A composition comprising a fungicidally effective amount of at least one cycloalkylcarboxamide of the formula I as claimed in any of claims 1-9 and, if appropriate, an inert liquid and/or solid carrier and, if appropriate, at least one surfactant.

11. A method for controlling harmful fungi, which comprises treating the harmful fungi, their habitat or the plants, areas, materials or spaces to be kept free from them with an effective amount of a cycloalkylcarboxamide of the formula I as claimed in any of claims 1-9.

12. The use of a cycloalkylcarboxamide of the formula I as claimed in any of claims 1-9 for controlling harmful fungi.

## Revendications

1. Cycloalkylcarboxamides de formule générale Ia 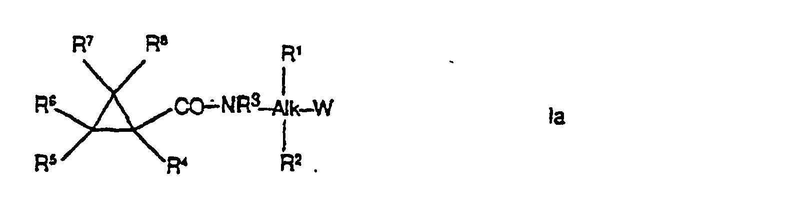 dans laquelle les symboles ont les significations suivantes Alk représente un groupe alkylène à chaîne droite ou ramifiée en C1-C6 ;
R¹ représente un groupe alkyle en C1-C6 ou alcényle en C2-C6, ces groupes pouvant être halogénés en totalité ou en partie et/ou pouvant porter un ou deux des substituants suivants : alcoxy en C1-C4, halogénoalcoxy en C1-C4, alkylthio en C1-C4, (alcoxy en C1-C4 )carbonyle, cycloalkyle en C3-C6 et phényle, le groupe phényle pouvant être halogéné en totalité ou en partie et/ou pouvant porter un à trois substituants suivants : nitro, cyano, alkyle en C1-C4, halogénoalkyle en C1-C4, alcoxy en C1-C4, halogénoalcoxy en C1-C4, alkylthio en C1-C4, cycloalkyle en C3-C6 ou hétérocyclyle ;
R², R³ : l'hydrogène, des groupes alkyle en C1-C6 ou alcényle en C2-C6, ces groupes pouvant être halogénés en totalité ou en partie ;
W : un système bicyclique condensé dont chaque cycle contient six atomes cycliques, un ou deux atomes de carbone cycliques pouvant être remplacés par des atomes d'azote, et ces systèmes cycliques pouvant porter un à trois des substituants suivants : nitro, halogéno, cyano, alkyle en C1-C4, halogénoalkyle en C1-C4, alcoxy en C1-C4, halogénoalcoxy en C1-C4, alkylthio en C1-C4, cycloalkyle en C3-C6 et (alcoxy en C1-C4)carbonyle ; R⁴ : l'hydrogène, un halogène, un groupe cyano, alkyle en C1-C6, alcényle en C2-C6, alcoxy en C1-C6 ou alkylthio en C1-C6, ces groupes pouvant être halogénés en totalité ou en partie et/ou pouvant porter un ou deux des substituants suivants alcoxy en C1-C4, halogénoalcoxy en C1-C4, alkylthio en C1-C4, (alcoxy en C1-C4)carbonyle, cycloalkyle en C3-C6, (alcoxy en C1-C4)carbonylamino;
R⁵ : l'hydrogène, un halogène, un groupe cyano, alkyle en C1-C6, alcényle en C2-C6, alcoxy en C1-C6 ou alkylthio en C1-C6, ces groupes pouvant être halogénés en totalité ou en partie et/ou pouvant porter un ou deux des substituants suivants alcoxy en C1-C4, halogénoalcoxy en C1-C4, alkylthio en C1-C4, (alcoxy en C1-C4)carbonyle, cycloalkyle en C3-C6, (alcoxy en C1-C4)carbonylamino ;
R⁶ : l'hydrogène, un halogène ou un groupe alkyle en C1-C6 ;
R⁷ : l'hydrogène, un halogène ou un groupe alkyle en C1-C6 ;
R⁸ : l'hydrogène, un halogène ou un groupe alkyle en C1-C6,
et leurs sels aptes aux applications agricoles.

2. Cycloalkylcarboxamides de formule générale Ia selon la revendication 1 pour lesquels le cycle cyclopropyle peut porter un à cinq substituants R⁴ à R⁸ choisis parmi les halogènes, les groupes cyano et les groupes alkyle en C1-C3.

3. Cycloalkylcarboxamides selon la revendication 1 pour lesquels R⁴ représente un halogène, un groupe cyano, alkyle en C1-C6, (alcoxy en C1-C4)carbonylamino, alcoxy en C1-C4 ou alkylthio en C1-C4.

4. Cycloalkylcarboxamides selon l'une des revendications 1 à 3 pour lesquels R⁵ représente l'hydrogène ou un groupe alkyle en C1-C6.

5. Cycloalkylcarboxamides selon l'une des revendications 1 à 4 pour lesquels R⁶ représente l'hydrogène ou un groupe alkyle en C1-C6.

6. Cycloalkylcarboxamides selon l'une des revendications 1 à 5 pour lesquels R⁷ représente l'hydrogène, le chlore ou un groupe alkyle en C1-C6.

7. Cycloalkylcarboxamides selon l'une des revendications 1 à 6 pour lesquels R⁸ représente l'hydrogène, le chlore ou un groupe alkyle en C1-C6.

8. Cycloalkylcarboxamides selon l'une des revendications 1 à 7 pour lesquels R¹ représente un groupe alkyle en C1-C6 et R² l'hydrogène ou un groupe alkyle en C1-C6.

9. Cycloalkylcarboxamides selon l'une des revendications 1 à 8 pour lesquels Alk représente un groupe méthylène.

10. Produit contenant une quantité efficace pour combattre les mycètes nuisibles d'au moins un cycloalkylcarboxamide de formule I selon l'une des revendications 1 à 9 et le cas échéant un véhicule liquide et/ou solide inerte ainsi que, le cas échéant, au moins une substance tensio-active.

11. Procédé pour combattre les mycètes nuisibles, **caractérisé en ce que** l'on traite les mycètes nuisibles, leur habitat, les végétaux, aires, matériaux ou locaux qu'on veut protéger contre les mycètes par une quantité efficace d'un cycloalkylalcanecarboxamide de formule I selon l'une des revendications 1 à 9.

12. Utilisation d'un cycloalkylalcanecarboxamide de formule générale I selon l'une des revendications 1 à 9 pour la lutte contre les mycètes nuisibles.
